Europäisches Patentamt

European Patent Office

Office européen des brevets

(19)

(11) Veröffentlichungsnummer: **0 033 437**
**A1**

(12) # EUROPÄISCHE PATENTANMELDUNG

(21) Anmeldenummer: **81100141.1**

(22) Anmeldetag: **10.01.81**

(51) Int. Cl.³: **G 01 N 33/54**
**G 01 N 33/58**

---

(30) Priorität: **02.02.80 DE 3003836**

(43) Veröffentlichungstag der Anmeldung:
**12.08.81 Patentblatt 81/32**

(84) Benannte Vertragsstaaten:
**AT BE CH DE FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Rieke, Erwin, Dr.**
**Hermannstrasse 12**
**D-6104 Seeheim-Jugenheim 1(DE)**

(72) Erfinder: **Würzburg, Uwe, Dr.**
**Dessauerstrasse 12**
**D-6110 Dieburg(DE)**

(72) Erfinder: **Hennrich, Norbert, Dr.**
**Haydnweg 14**
**D-6100 Darmstadt(DE)**

---

(54) **Verfahren und Mittel zur Bestimmung einer Komponente aus einer Gruppe bestehend aus spezifisch bindende Rezeptoren und Substanzen, die von diesen Rezeptoren spezifisch gebunden werden können.**

(57) Die Erfindung betrifft Verfahren und Mittel zur Bestimmung einer Komponente aus einer Gruppe bestehend aus spezifisch bindenden Rezeptoren und Substanzen, die von diesen Rezeptoren spezifisch gebunden werden können, durch Messung der Aktivität von durch Inkubation daran gebundenen Enzymen, die dadurch gekennzeichnet sind, daß man eine der Komponenten nach Immobilisierung an einem wasserunlöslichen Träger mit der jeweils anderen Komponente inkubiert, anschließend nach üblichem Waschen mit an Saccharid bindenden Enzymen inkubiert und die Enzymaktivität bestimmt. Als an Saccharid bindende Enzyme können solche mit Lectineigenschaften bzw. Lectine mit enzymatischer Aktivität verwendet werden.

EP 0 033 437 A1

Merck Patent Gesellschaft
mit beschränkter Haftung
Darmstadt

Verfahren und Mittel zur Bestimmung
einer Komponente aus einer Gruppe bestehend aus spezifisch bindenden Rezeptoren
und Substanzen, die von diesen Rezeptoren
spezifisch gebunden werden können

Die Erfindung betrifft Verfahren und Mittel zur Bestimmung einer Komponente aus einer Gruppe bestehend aus spezifisch bindenden Rezeptoren und Substanzen, die von
diesen Rezeptoren spezifisch gebunden werden können,
insbesondere Verfahren und Mittel zur immunologischen
Bestimmung von Antikörpern, Antigenen und Haptenen
in Lösung über die vermittelnde Wirkung einer
enzymatischen Aktivität.

Für die bisher beschriebenen Enzymimmunoassays werden
Kupplungsprodukte aus einem Markerenzym und einem Antikörper bzw. einem Antigen benötigt (DAS 2.206.103). Mit
Hilfe dieser Antikörper-Enzym-Konjugate werden die über
ein Immunosorbens gebundenen Antigene markiert. Die gebundene Enzymaktivität ist ein Maß für die Menge an vorhandenem Antigen. Für Kompetitionsverfahren müssen z. B.

GSPHA18 A

Kupplungsprodukte hergestellt werden, die mit dem Antigen der Probe um die Bindung an das Immunosorbens konkurrieren. Hier verhält sich die gebundene Enzymaktivität umgekehrt proportional zur Menge an vorhandenem Antigen.

Die Notwendigkeit, Antikörper- bzw. Antigen-Enzym-Konjugate herstellen zu müssen, beinhaltet eine Reihe wesentlicher Nachteile: Durch die chemische Verknüpfung eines Antikörpers (Antigens) mit einem Enzym wird der Antikörper (Antigen) in seinem Bindungsverhalten gestört, d. h. die Bindungsaffinität nimmt ab; durch die chemische Verknüpfung eines Enzyms mit einem Antikörper (Antigen) wird die enzymatische Aktivität wesentlich vermindert; die Stabilität der Konjugate ist geringer als die Stabilität der Einzelsubstanzen; die Konjugate stellen sehr heterogene Verbindungen mit breitem Molekulargewichtsspektrum dar, wodurch eine reproduzierbare Herstellung außerordentlich erschwert wird; die enzymatische Aktivität wird mit den an eine feste Phase gebundenen Konjugaten bestimmt, wobei die Diffusion von Substraten und Produkten den enzymatischen Umsatz beeinflußt.

Der vorliegenden Erfindung liegt die Aufgabe zugrunde, Verfahren und Mittel zur Bestimmung von Antikörpern, Antigenen und Haptenen zur Verfügung zu stellen, mit denen die geschilderten Nachteile vermieden werden können.

Erfindungsgemäß wurde diese Aufgabe dadurch gelöst, daß man anstelle eines Kupplungsprodukts aus einem Markerenzym und einem Antikörper bzw. Antigen ein Enzym bzw. ein enzymatisch aktives Lectin verwendet, das neben der Substratbindungsstelle weitere Bindungsstellen für den Antikörper bzw. das Antigen besitzt.

GSPHA18 A

Gegenstand der Erfindung ist ein Verfahren zur Bestimmung einer Komponente aus einer Gruppe bestehend aus spezifisch bindenden Rezeptoren und Substanzen, die von diesen Rezeptoren spezifisch gebunden werden können, durch Messung der Aktivität von durch Inkubation daran gebundenen Enzymen, das dadurch gekennzeichnet ist, daß man eine der Komponenten nach Immobilisierung an einem wasserunlöslichen Träger mit der jeweils anderen Komponente inkubiert, anschließend nach üblichem Waschen mit an Saccharid bindenden Enzymen inkubiert und die Enzymaktivität bestimmt.

Als immobilisierte Komponente kann eine modifizierte Komponente verwendet werden, deren Saccharidgruppierung chemisch oder enzymatisch so verändert ist, daß sie keine an Saccharid bindenden Enzyme mehr bindet.

In einer Variante des Verfahrens kann man eine an einem wasserunlöslichen Träger immobilisierte Komponente gleichzeitig oder nacheinander mit der jeweils anderen Komponente und einem Kupplungsprodukt aus der jeweils anderen Komponente und einem Saccharid-haltigen Molekül inkubieren. In einer weiteren Variante kann man eine an einem wasserunlöslichen Träger immobilisierte Komponente mit der jeweils anderen, zu bestimmenden Komponente inkubieren und anschließend nach üblichem Waschen mit einer weiteren Saccharid-haltigen, an die zu bestimmende Komponente bindenden Komponente inkubieren.

Ferner betrifft die Erfindung ein Mittel zur Durchführung dieses Verfahrens, enthaltend einen wasserunlöslichen Träger, an den eine der Komponenten oder ein Komplex aus den Komponenten, wovon eine der Komponenten aus einem Kupplungsprodukt mit einem Saccharid be-

GSPHA18 A

steht, gebunden ist - gegebenenfalls ein Kupplungsprodukt aus einer der Komponenten und einem Saccharid-haltigen Molekül - ein an Saccharid bindendes Enzym und Substrate zur Bestimmung der Enzymaktivität.

Überraschenderweise hat sich gezeigt, daß das erfindungsgemäße Verfahren exakter und schneller arbeitet als die herkömmlichen Enzymimmunoassays. Es kann auf die Herstellung von Antikörper- bzw. Antigen-Enzym-Konjugaten verzichtet werden, wodurch eine Störung der immunologischen Reaktion ausgeschaltet wird; das Enzym muß nicht modifiziert werden und kann daher bis zu seiner Verwendung in einer für das Enzym bevorzugten Form gelagert werden; die Reagenzien können reproduzierbar und standardisierbar hergestellt werden; da das Enzym durch das Substrat wieder aus der Bindung mit dem Antikörper (Antigen) abgelöst wird, verläuft die enzymatische Reaktion in Lösung und nicht auf der Röhrchenoberfläche; die Bindung des Enzyms an Antikörper verläuft außerordentlich schnell (bereits nach 15 - 60 Minuten kann die Inkubation abgebrochen werden); die für ein Kompetitionsverfahren herzustellenden Antigen-Saccharid-Konjugate haben eine gegenüber Antigen-Enzym-Konjugaten wesentlich höhere Haltbarkeit, außerdem wird das Antigen durch den Einbau von Zuckerresten nur unwesentlich modifiziert.

Mit dem erfindungsgemäßen Verfahren können prinzipiell alle organischen Substanzen, zu denen ein Bindungspartner existiert, quantitativ bestimmt werden. Die bevorzugte Ausführung gestaltet sich besonders einfach, wenn die zu bestimmende Substanz in ihrem molekularen Aufbau Saccharidgruppierungen aufweist, die von einem mit Saccharid reagierenden Enzym bzw. einem Lectin mit enzymatischer Aktivität erkannt und gebunden werden können. Dies ist bei Antikörpern, bei einer Vielzahl von Antigenen und bei einigen Haptenen der Fall.

GSPHA18 A

Zur Durchführung des erfindungsgemäßen Verfahrens wird die Probelösung, die die zu bestimmende Substanz enthält, mit dem an einem wasserunlöslichen Träger immobilisierten Bindungspartner, der oder an den die zu bestimmende Substanz spezifisch bindet, inkubiert. Während der Inkubation bindet eine der Konzentration der Substanz entsprechende Menge über ihren Bindungspartner an das Trägermaterial. Nach dem Entfernen der Probelösung wird das Trägermaterial gewaschen, um unspezifisch anhaftende Substanzen zu entfernen. Anschließend wird dieses Trägermaterial mit einem mit einer Saccharidgruppierung reagierenden Enzym für eine kurze Zeit in Kontakt gebracht, wobei das Enzym an die zu bestimmende Substanz gebunden wird. Nach dem Auswaschen von nicht gebundenem Enzym wird dem Trägermaterial eine Lösung mit einem Enzymsubstrat zugesetzt. Die Enzymaktivität kann dann photometrisch gemessen werden und ist direkt ein Maß für die Menge der zu bestimmenden Substanz in der Probelösung.

Dieses einfache Verfahren läßt sich bei Verwendung von immobilisierten Antikörpern oder sonstigen Rezeptoren zur Bestimmung von Saccharid-haltigen Antigenen und Haptenen oder bei immobilisierten Antigenen oder Haptenen zur Bestimmung ihrer entsprechenden Antikörper oder Saccharid-haltigen Rezeptoren verwenden.

Weist auch der am wasserunlöslichen Träger immobilisierte Bindungspartner Saccharidgruppierungen auf, die vom eingesetzten Enzym erkannt werden können, so können diese, falls erforderlich, zweckmäßigerweise vor dem Immobilisieren chemisch oder enzymatisch abgebaut werden.

GSPHA18 A

Weist die zu bestimmende Substanz keine geeignete Saccharidstruktur auf, die die Bindung eines mit Saccharid reagierenden Enzyms ermöglicht, so kann als zusätzliches Reagenz eine bekannte Menge der zu bestimmenden Substanz verwendet werden, die jedoch chemisch oder enzymatisch mit einem geeigneten Saccharid verknüpft worden ist und durch diese Markierung von dem betreffenden Enzym erkannt werden kann. Da man dieses Konjugat in der obigen Weise bestimmen kann, ist es leicht möglich, die zu bestimmende Substanz durch ein Kompetitionsverfahren quantitativ zu bestimmen. Man kann dabei so vorgehen, daß man die zu bestimmende Substanz aus der Probe und die markierte Substanz gleichzeitig oder mit einer zeitlichen Verzögerung mit einer limitierten Menge des am wasserunlöslichen Träger immobilisierten Bindungspartners inkubiert. Dabei konkurrieren die zu bestimmende Substanz und die markierte Substanz um die Bindung an dem gemeinsamen Bindungspartner. Liegt die zu bestimmende Substanz in einer hohen Konzentration vor, so wird nur wenig der markierten Substanz an das Trägermaterial binden, d. h. die nach der Inkubation mit dem saccharidbindenden Enzym am Trägermaterial anhaftende Enzymaktivität ist bei diesem Verfahren umgekehrt proportional zur Konzentration der zu bestimmenden Substanz in der Probelösung.

Eine Variante dieses Verfahrens besteht darin, daß man den Bindungspartner für die zu bestimmende Substanz am wasserunlöslichen Träger immobilisiert und ihn dann mit einem Überschuß an markierter Substanz inkubiert. Hierdurch sättigt man den Bindungspartner mit der markierten Substanz ab, d. h. am Trägermaterial liegen jetzt präformierte Komplexe aus dem Bindungspartner und der markierten Substanz vor. Der Test besteht dann lediglich in der Inkubation dieses Trägermaterials mit der Probelösung. Je nach der Konzentration der zu bestimmenden Substanz wird die markierte Substanz wieder aus dem Komplex verdrängt und der Nachweis kann dann über die Bindung des saccharidbindenden Enzyms erfolgen.

Die Markierung von Haptenen und Antigenen mit einem geeigneten Saccharid kann durch direkte chemische oder enzymatische Kupplung erfolgen. Andererseits kann die zu bestimmende Substanz aber auch an Moleküle gekuppelt werden, die in ihrer Molekülstruktur u.a. die geeignete Saccharidgruppierung aufweisen. Solche Moleküle können ein Molekulargewicht von einigen 100 bis zu 1 Million oder mehr aufweisen; vorzugsweise liegt das Molekulargewicht unter 600 000, insbesondere unter 300 000. Solche Moleküle sind Glycoproteine, Polysaccharide, glycosylierte Proteine oder kleinere Moleküle, die neben der geeigneten Saccharidgruppierung noch Gruppen für die Kupplung der zu bestimmenden Substanz aufweisen. Die aus der Kupplung resultierenden Konjugate konkurrieren dann wie oben beschrieben um die Bindung an den immobilisierten Bindungspartner. Der weitere Testverlauf entspricht dann dem beim Kompetitionsverfahren beschriebenen.

Eine weitere erfindungsgemäße Möglichkeit zur Bestimmung von Substanzen, die keine geeignete Saccharidstruktur aufweisen, besteht in der Verwendung eines zusätzlichen löslichen Bindungspartners. Hierbei wird die Probe mit der zu bestimmenden Substanz mit dem immobilisierten Bindungspartner inkubiert, wobei ein der Konzentration der Substanz entsprechender Anteil über den Bindungspartner an den Träger bindet. Nach dem Auswaschen der

GSPHA18 A

Probelösung wird der lösliche Bindungspartner zugesetzt. Dieser lösliche Bindungspartner kann dem immobilisierten Bindungspartner entsprechen oder eine andere Zusammensetzung aufweisen. Entscheidend ist nur, daß er eine geeignete Saccharidgruppierung aufweist und mit ausreichender Affinität an die über den immobilisierten Bindungspartner am Trägermaterial gebundene Substanz bindet. Die Bindung des löslichen Bindungspartners kann dann wieder durch ein saccharidbindendes Enzym in bekannter Weise bestimmt werden. Die am Trägermaterial anhaftende Aktivität ist proportional zur Konzentration der zu bestimmenden Substanz.

Die Voraussetzung für die oben beschriebene Verfahrensvariante besteht darin, daß die zu bestimmende Substanz mehrere Bindungsstellen aufweist, damit sowohl der immobilisierte als auch der lösliche Bindungspartner binden können. Weist die zu bestimmende Substanz jedoch nur eine Bindungsstelle auf, so ist es möglich, mehrere Moleküle dieser Substanz chemisch an ein Trägermolekül zu binden, so daß Konjugate entstehen, die mehrere Bindungspartner binden können. In diesem Fall muß dann zuerst ein Kompetitionsverfahren durchgeführt werden, d. h. das Konjugat und die zu bestimmende Substanz konkurrieren um die Bindung an dem immobilisierten Bindungspartner. Die Menge an gebundenem Konjugat hängt von der Konzentration der zu bestimmenden Substanz in der Probelösung ab. An das gebundene Konjugat kann dann der lösliche Bindungspartner binden, der dann wiederum für die Bindung des saccharidbindenden Enzyms zur Verfügung steht. Die am Träger anhaftende Enzymaktivität ist umgekehrt proportional zur Konzentration der zu bestimmenden Substanz in der Probe.

GSPHA18 A

Zur Durchführung des erfindungsgemäßen Verfahrens sind Enzyme mit Lectineigenschaften bzw. Lectine mit enzymatischer Aktivität geeignet, die mit Sacchariden reagieren, vorzugsweise Galactoseoxidase, Galactosidase und/oder Mannosidase. Als besonders geeignet hat sich Galactoseoxidase aus Dactylum dendroides erwiesen.

Als wasserunlösliche Träger werden vorzugsweise Kunststoffgefäße verwendet, insbesondere Teströhrchen aus Polystyrol oder Polypropylen.

Eine Bestimmung von Antigenen wird im einfachsten Fall so durchgeführt, daß zunächst Polystyrolröhrchen mit Antikörpern beschichtet werden. Die Beschichtung erfolgt ca. 3 bis 24 Stunden lang bei Raumtemperatur in einem geeigneten Puffer, z. B. Boratpuffer, Phosphatpuffer, Carbonatpuffer oder Tris-Puffer in einer Konzentration von 0,01 - 0,5 Mol/l und bei pH-Werten von 7 - 10, vorzugsweise in einem 0,1 M Boratpuffer vom pH-Wert 8,4. Danach werden die Röhrchen entleert und mit einem detergenzhaltigen Puffer gewaschen. Vorzugsweise wird dazu ein Phosphatpuffer verwendet, der etwa 0,01 bis 0,1 % eines oder mehrerer Detergenzien wie Polyoxyäthylensorbitanmonooleat, Polyoxyäthylenäther höherer Alkohole usw. enthält.

Die mit Antikörper beschichteten Röhrchen werden dann mit den zu bestimmenden Antigenen inkubiert. Diese Inkubation erfolgt ca. 2 bis 24 Stunden lang bei Raumtemperatur in den genannten Puffern, vorzugsweise in einem 0,05 bis 0,2 M Phosphatpuffer vom pH-Wert 7,5 dem etwa 0,5 - 2 % Ovalbumin oder ein anderes Protein zugesetzt werden. Anschließend werden die Röhrchen wieder entleert und mit einem detergenzhaltigen Puffer gewaschen.

Die anschließende Inkubation mit einem Enzym, das Lectineigenschaften hat oder mit einem Lectin mit enzymatischer Aktivität, erfolgt in einem 0,05 bis 0,2 M Phosphatpuffer, vorzugsweise in einem 0,1 M Kaliumphosphatpuffer vom pH-Wert 7,2. Als an Saccharid bindende Enzyme mit Lectineigenschaften eignen sich vor allem Galactoseoxidase, Galactosidase und Mannosidase, als Lectine mit enzymatischer Aktivität werden Phytohämagglutinine aus Limabohne oder Mungobohne bevorzugt (J.Biol.Chem. 253, 7791 (1978)). Nach 15 - 60 Minuten werden die Röhrchen wieder entleert und mit dem oben beschriebenen detergenzhaltigen Puffer gewaschen.

Bei Verwendung von Galactoseoxidase als Enzym erfolgt die Nachweisreaktion mit Galactose als Substrat. Die Röhrchen werden mit einer Reaktionslösung versetzt, die z. B. in einem 0,1 M Phosphatpuffer Galactose, Phenol, Aminoantipyrin und Peroxidase enthält. Nach etwa einer Stunde wird die Extinktion der Lösung bei 492 nm gemessen. Es können auch andere Reaktionslösungen zur Wasserstoffperoxidbestimmung eingesetzt werden, wie z. B. ein 0,2 M Phosphatpuffer, der Galactose, Kaliumjodid, Ammoniummolybdat und Äthylendinitrilotetraessigsäure enthält. Die entstehende Färbung wird dann bei 350 nm gemessen.

Beispiel 1

Bestimmung von anti-BSA Antikörpern

a) Herstellung von BSA-beschichteten Röhrchen Polystyrolröhrchen wurden adsorptiv mit Rinderserumalbumin (BSA) beschichtet. Hierfür wurden die Röhrchen mit 1 ml einer Lösung von 10 µg BSA

GSPHA18 A

pro ml 0,1 M Boratpuffer (pH 8,4) gefüllt und 20
Stunden bei Raumtemperatur stehengelassen. Danach
wurden die Röhrchen entleert und zweimal mit einem
Phosphatpuffer gewaschen, der 0,05 % Polyoxyäthylensorbitanmonooleat enthielt.

b)    Inkubation mit anti-BSA Eine Lösung von anti-BSA (IgG aus Kaninchen) wurde
durch Zusatz von 0,1 M Phosphatpuffer (pH 7,5) (mit
1 % Ovalbumin) auf Konzentrationen von 1 bis
10 000 ng IgG/ml verdünnt. Jeweils 1 ml dieser Lösungen wurden in die mit BSA beschichteten Röhrchen
gegeben und 18 Stunden bei Raumtemperatur stehengelassen. Danach wurden die Röhrchen entleert und
zweimal mit detergenzhaltigem Phosphatpuffer
gewaschen.

c)    Inkubation mit Galactoseoxidase 450 Einheiten Galactoseoxidase wurden in 2 ml 0,1 M
Kaliumphosphatpuffer (pH 7,2) gelöst und vor Gebrauch mit dem gleichen Puffer 1 : 30 verdünnt. Jeweils 1 ml dieser Lösung wurde den Röhrchen zugesetzt. Nach 15 - 60 Minuten wurden die Röhrchen
entleert und einmal mit detergenzhaltigem Phosphatpuffer gewaschen.

d)    Nachweisreaktion Die Röhrchen wurden mit 1 ml einer Reaktionslösung
folgender Zusammensetzung versetzt: 100 ml einer
Lösung von 2,35 g Phenol und 50 g Galactose pro
Liter 0,1 M Natriumphosphatpuffer (pH 7,3) wurden
mit 1 ml einer Lösung von 0,2 M Aminoantipyrin versetzt. Hierzu wurden 5 ml einer Lösung
von 1 mg/ml Peroxidase gegeben. Nach 60 Minuten wurde die Extinktion der Lösung bei 492 nm im Photometer gemessen. Wie die nachstehende Tabelle zeigt, korreliert die Extinktion mit der Menge an zugesetzten Antikörpern gegen BSA.

| Menge an anti-BSA [ng/ml] | Extinktion bei 492 nm [OD] |
|---|---|
| 0 | 0,012 |
| 1 | 0,035 |
| 10 | 0,063 |
| 100 | 0,080 |
| 1000 | 0,122 |
| 10000 | 0,268 |

Beispiel 2

Bestimmung von Antigenen, die Zuckerreste enthalten: human-IgG (h-IgG)

a) Beschichtung von Röhrchen mit Antikörpern gegen h-IgG aus Schaf (a-h-IgG)
Polystyrolröhrchen wurden adsorptiv mit Antikörpern gegen h-IgG (a-h-IgG) beschichtet. Hierfür wurden die Röhrchen mit 1 ml einer Lösung von 10 µg a-h-IgG pro ml 0,1 M Boratpuffer (pH 8,4) gefüllt und 20 Stunden bei Raumtemperatur stehengelassen. Danach wurden die Röhrchen entleert und zweimal mit detergenzhaltigem Phosphatpuffer gewaschen.

b) Inkubation mit h-IgG Eine Lösung von h-IgG wurde durch Zusatz von 0,1 M Phosphatpuffer (mit 1 % Ovalbumin) auf Konzentrationen von 1 - 10 ng/ml verdünnt. Jeweils 1 ml dieser Lösungen wurde in die mit a-h-IgG beschichteten Röhrchen gegeben und 18 Stunden bei Raumtemperatur stehengelassen. Danach wurden die Röhrchen entleert und zweimal mit detergenzhaltigem Phosphatpuffer gewaschen.

c) Inkubation mit Galactoseoxidase 450 Einheiten Galactoseoxidase wurden in 2 ml 0,1 M Kaliumphosphatpuffer (pH 7,2) gelöst und vor Gebrauch mit dem gleichen Puffer 1 : 50 verdünnt. Jeweils 1 ml dieser Lösung wurde den Röhrchen zugesetzt. Nach 15 - 60 Minuten wurden die Röhrchen entleert und und einmal mit detergenzhaltigem Phosphatpuffer gewaschen.

d) Nachweisreaktion Die Röhrchen wurden mit 1 ml einer Reaktionslösung analog Beispiel 1 d versetzt. Nach 60 Minuten wurde die Extinktion der Lösung bei 492 nm gegen den Nullwert im Photometer gemessen. Die Extinktion korreliert mit der Menge an zugesetztem h-IgG, wie die nachstehende Tabelle zeigt.

| Menge an h-IgG [µg/ml] | Extinktion bei 492 nm [OD] |
|---|---|
| 0 | 0,213 |
| 1 | 0,220 |
| 2 | 0,225 |
| 4 | 0,232 |
| 6 | 0,236 |
| 8 | 0,242 |
| 10 | 0,271 |

GSPHA18 A

Beispiel 3

Bestimmung von Antigenen nach Markierung mit Zuckerresten: Mit Melibiose markiertes Rinderserumalbumin
(BSA-Melibiose)

a)   Beschichtung von Röhrchen mit Antikörpern gegen
     BSA aus Kaninchen (a-BSA) Polystyrolröhrchen wurden adsorptiv mit Antikörpern
gegen BSA (a-BSA) beschichtet. Hierfür wurden die
Röhrchen mit 1 ml einer Lösung von 10 µg a-BSA
pro ml Boratpuffer (pH 8,4) gefüllt und 20 Stunden
bei Raumtemperatur stehengelassen. Danach wurden
die Röhrchen entleert und zweimal mit detergenzhaltigem Phosphatpuffer gewaschen.

b)   Herstellung von BSA-Melibiose-Konjugaten 68 mg BSA, 100 mg Melibiose und 10 mg Natriumcyanoborhydrid wurden in 5 ml 0,2 M Kaliumphosphatpuffer
(pH 8,0) gelöst und bei 37°C 48 Stunden stehengelassen. Die Lösung wurde zur Abtrennung der überschüssigen Melibiose über Sephadex G-25 in 0,1 M
Kaliumphosphatpuffer (pH 7,0) chromatographiert.

c)   Inkubation mit BSA-Melibiose Eine Lösung von BSA-Melibiose wurde durch Zusatz
von Phosphatpuffer (mit 1 % Ovalbumin) auf Konzentrationen von 0,1 - 10 ng/ml verdünnt. Jeweils
1 ml dieser Lösungen wurde in die mit a-BSA beschichteten Röhrchen gegeben und 18 Stunden bei
Raumtemperatur stehengelassen. Danach wurden die

GSPHA18 A

Röhrchen entleert und zweimal mit detergenzhaltigem Phosphatpuffer gewaschen.

Die Inkubation mit Galactoseoxidase erfolgte analog Beispiel 2 c.

d) Nachweisreaktion Die Röhrchen wurden mit 1 ml einer Reaktionslösung analog Beispiel 1 d versetzt. Nach 60 Minuten wurde die Extinktion der Lösung bei 492 nm gegen den Nullwert im Photometer gemessen. Die Extinktion korreliert mit der Menge an zugesetztem BSA-Melibiose-Konjugat, wie die nachstehende Tabelle zeigt.

| Menge an BSA-Melibiose [µg/ml] | Extinktion bei 492 nm [OD] |
|---|---|
| 0,1 | 0,237 |
| 1,0 | 0,294 |
| 10,0 | 0,329 |

In analoger Weise können Thyroxin oder ein Konjugat aus Thyroxin und BSA mit Melibiose markiert werden. Diese Konjugate oder auch ein Konjugat aus Thyroxin und einem $\gamma$-Globulin können unter Verwendung von mit anti-Thyroxin beschichteten Röhrchen quantitativ bestimmt werden.

Beispiel 4

Bindung der Galactoseoxidase in einer Sandwich-
Reaktion: Bestimmung von BSA

Die Beschichtung von Röhrchen mit Antikörpern gegen BSA
aus Kaninchen (a-BSA) erfolgte analog Beispiel 2 a.

a)    Inkubation mit BSA Eine Lösung von BSA wurde durch Zusatz von Phosphatpuffer (mit 1 % Ovalbumin) auf Konzentrationen
von 1 - 10 000 ng/ml verdünnt. Jeweils 1 ml dieser
Lösungen wurde in die mit a-BSA beschichteten
Röhrchen gegeben und 18 Stunden bei Raumtemperatur
stehengelassen. Danach wurden die Röhrchen entleert und zweimal mit detergenzhaltigem Phosphatpuffer gewaschen.

b)    Inkubation mit a-BSA Die Röhrchen wurden 3 Stunden mit je 1 ml einer
Lösung von 1 ng a-BSA in 10 mM Tris-Puffer inkubiert und dann entleert. Es wurde zweimal mit
detergenzhaltigem Phosphatpuffer gewaschen.

Die Inkubation mit Galactoseoxidase erfolgte
analog Beispiel 2 c.

c)    Nachweisreaktion Die Röhrchen wurden mit 1 ml einer Reaktionslösung analog Beispiel 1 d versetzt. Nach 60 Minuten wurde die Extinktion der Lösung bei 492 nm

GSPHA18 A

gegen den Nullwert im Photometer gemessen. Die Extinktion korreliert mit der Menge an zugesetztem BSA, wie die nachstehende Tabelle zeigt.

| Menge an BSA [µg/ml] | Extinktion bei 492 nm [OD] |
|---|---|
| 1 | 0,396 |
| 10 | 0,419 |
| 100 | 0,465 |
| 1 000 | 0,493 |
| 10 000 | 0,543 |

Beispiel 5

Bindung der Galactoseoxidase an Antikörper und modifizierte Antikörper

a) Beseitigung der Oligosaccharide bei Glycoproteinen 4 mg anti-human-IgG aus Schaf (a-h-IgG) wurden in 1 ml dest. Wasser gelöst und mit 0,2 ml einer frischen 0,1 M Natriumperjodatlösung 20 Minuten bei Raumtemperatur gerührt. Danach wurde die Lösung gegen 1 mM Natriumacetatpuffer (pH 4,4) bei 4°C über Nacht dialysiert. Nach Zugabe von 1 ml einer 0,1 M Lysinlösung in 0,2 M Natriumcarbonatpuffer (pH 9,5) wurde 2 Stunden bei Raumtemperatur gerührt und dann 0,1 ml einer Lösung von 4 mg Natriumborhydrid pro ml zugefügt. Nach 2 Stunden wurde gegen Boratpuffer (pH 8,4) dialysiert.

b) Beschichtung von Röhrchen mit a-h-IgG und modifiziertem a-h-IgG Von den Antikörpern wurden Verdünnungen zu 0,1, 1,0 und 10 µg pro ml in Boratpuffer (pH 8,4) hergestellt. Polystyrolröhrchen wurden mit je 1 ml der jeweiligen Lösung über Nacht stehengelassen. Danach wurden die Röhrchen entleert und zweimal mit detergenzhaltigem Phosphatpuffer gewaschen.

Die Inkubation mit Galactoseoxidase erfolgte analog Beispiel 2 c.

c) Nachweisreaktion Die Röhrchen wurden mit 1 ml einer Reaktionslösung analog Beispiel 1 d versetzt. Nach 60 Minuten wurde die Extinktion der Lösung bei 492 nm gegen den Nullwert im Photometer gemessen. Die Extinktion korreliert mit der Menge an a-h-IgG im Beschichtungspuffer, wie die nachstehende Tabelle zeigt. Oxidiertes a-h-IgG zeigte nur noch eine sehr geringe Bindungsaffinität zu Galactoseoxidase.

| a-h-IgG im Beschichtungspuffer [µg/ml] | Extinktion bei 492 nm [OD] | modifiziertes a-h-IgG im Beschichtungspuffer [µg/ml] | Extinktion bei 492 nm [OD] |
|---|---|---|---|
| 0,1 | 0,04 | 0,1 | 0,01 |
| 1,0 | 0,47 | 1,0 | 0,02 |
| 10,0 | 0,53 | 10,0 | 0,03 |

GSPHA18 A

0033437

Beispiel 6

Bestimmung von Antigenen, die Zuckerreste enthalten:
human-IgE (h-IgE)

a) Beschichtung von Röhrchen mit Antikörpern gegen h-IgE aus Ziege (a-h-IgE) Polystyrolröhrchen wurden adsorptiv mit a-h-IgE beschichtet, das analog Beispiel 5 modifiziert worden war. Hierfür wurden die Röhrchen mit 1 ml einer Lösung von 0,1 µg a-h-IgE pro ml Boratpuffer (pH 8,4) gefüllt und 20 Stunden bei Raumtemperatur stehengelassen. Danach wurden die Röhrchen entleert und zweimal mit detergenzhaltigem Phosphatpuffer gewaschen.

b) Inkubation mit h-IgE Eine Lösung von h-IgE wurde durch Zusatz von Phosphatpuffer (mit 1 % Ovalbumin) auf Konzentrationen von 1 - 100 ng/ml verdünnt. Jeweils 1 ml dieser Lösungen wurde in die mit a-h-IgE beschichteten Röhrchen gegeben und 18 Stunden bei Raumtemperatur stehengelassen. Danach wurden die Röhrchen entleert und zweimal mit detergenzhaltigem Phosphatpuffer gewaschen.

Die Inkubation mit Galactoseoxidase erfolgte analog Beispiel 2 c.

c) Nachweisreaktion Die Röhrchen wurden mit 1 ml einer Reaktionslösung analog Beispiel 1 d versetzt. Nach 60 Minuten

GSPHA18 A

wurde die Extinktion der Lösung bei 492 nm gegen den Nullwert im Photometer gemessen. Die Extinktion korreliert mit der Menge an zugesetztem h-IgE, wie die nachstehende Tabelle zeigt.

| Menge an h-IgE [ng/ml] | Extinktion bei 492 nm [OD] |
|---|---|
| 1 | 0,054 |
| 10 | 0,069 |
| 100 | 0,081 |

Beispiel 7

Bestimmung von Antikörpern, die Zuckerreste enthalten, mit ß-Galactosidase: a-BSA

Die Beschichtung von Röhrchen mit BSA und die Inkubation mit anti-BSA erfolgte analog Beispiel 1 a und 1 b.

a)    Inkubation mit ß-Galactosidase

Eine Kristallsuspension von ß-Galactosidase mit 5 mg/ml wurde 1 : 500 mit 10 mM Tris-HCl-Puffer verdünnt, der 10 mM Magnesiumchlorid, 10 mM Mercaptoäthanol und 10 mM Natriumchlorid enthielt. Je 1 ml dieser Enzymlösung wurde in die Röhrchen gegeben. Nach 2 Stunden wurden die Röhrchen entleert und einmal mit 0,1 M Kaliumphosphatpuffer (pH 7,0) gewaschen.

b)    Nachweisreaktion Zu den Röhrchen wurde jeweils 1 ml einer Substratlösung gegeben, die sich wie folgt zusammensetzt:

15 ml 0,3 M Kaliumphosphatpuffer mit 3 mM Magnesiumchlorid, 4,5 ml 1 M Mercaptoäthanol, 7,5 ml 14 mM 2-Nitrophenyl-ß-D-galactopyranosid in 10 mM Tris-Acetat-Puffer mit 10 mM Magnesiumchlorid und 18 ml dest. Wasser. Nach 1 - 2 Stunden wurde die Extinktion der Lösung im Photometer bei 405 nm gemessen. Die Werte korrelieren mit der Menge an zugesetztem a-BSA, wie die nachstehende Tabelle zeigt.

| Menge an a-BSA [ng/ml] | Extinktion bei 405 nm [OD] |
|---|---|
| 1 | 0,14 |
| 10 | 0,17 |
| 100 | 0,22 |
| 1 000 | 0,25 |
| 10 000 | 0,33 |

Beispiel 8

Bestimmung von Choriongonadotropin (hCG)

a) Beschichtung von Röhrchen mit Antikörpern gegen Choriongonadotropin (a-hCG).

Polystyrolröhrchen wurden adsorptiv mit a-hCG beschichtet. Hierfür wurden die Röhrchen mit 1 ml einer Lösung von 10 µg a-hCG pro ml 0,1 M Carbonatpuffer (pH 9,0) gefüllt und 4 Stunden bei Raumtemperatur stehengelassen. Danach wurden die Röhrchen entleert und zweimal mit detergenzhaltigem Phosphatpuffer gewaschen.

b) Inkubation mit hCG Eine Lösung von hCG (3000 I.U./mg) wurde durch Zusatz von 0,1 M Phosphatpuffer (mit 1 % Ovalbumin) auf Konzentrationen von 1 bis 320 I.U./ml verdünnt.

- 22 -

0033437

Jeweils 1 ml dieser Lösungen wurde in die mit a-hCG beschichteten Röhrchen gegeben und 4 Stunden bei Raumtemperatur stehengelassen. Danach wurden die Röhrchen entleert und zweimal mit detergenzhaltigem Phosphatpuffer gewaschen.

c) Inkubation mit Galactoseoxidase 450 Einheiten Galactoseoxidase wurden in 2 ml 0,1 M Kaliumphosphatpuffer (pH 7,2) gelöst und vor Gebrauch mit 0,01 M 2-Morpholinäthanolsulfonsäure-Puffer (pH 5,0) 1:100 verdünnt. Jeweils 1 ml dieser Lösung wurde den Röhrchen zugesetzt. Nach 5-60 Minuten bei 4°C wurden die Röhrchen entleert und einmal mit dem gleichen Puffer gewaschen.

d) Nachweisreaktion Die Röhrchen wurden mit 1 ml einer Reaktionslösung analog Beispiel 1 d versetzt. Nach 10 Minuten wurde die Extinktion der Lösung bei 492 nm im Photometer gemessen.

Wie die nachstehende Tabelle zeigt, korreliert die Extinktion mit der Menge an zugesetztem hCG.

| Mengen an hCG [mI.U./ml] | Extinktion bei 492 nm [OD] |
|---|---|
| 0 | 0,020 |
| 1,25 | 0,026 |
| 12,5 | 0,050 |
| 125,0 | 0,100 |
| 313,0 | 0,174 |

Analoge Ergebnisse wurden erhalten, wenn anstelle von 2-Morpholinäthanolsulfonsäure als Verdünnungspuffer für die Galactoseoxidase (Schritt c) die folgenden Puffer verwendet wurden:
Acetat, Citrat, Tris, Glycin, Glycylglycin, N,N-Bis-
(2-hydroxy-äthyl)glycin, Piperazin-1,4-diäthansulfon-
säure.

Beispiel 9

Bestimmung von human-IgE (h-IgE)

a) Beschichtung von Röhrchen mit Antikörpern gegen
h-IgE aus Kaninchen (a-h-IgE) Polystyrolröhrchen wurden adsorptiv mit a-h-IgE beschichtet. Hierfür wurden die Röhrchen mit 1 ml
einer Lösung von 10 $\mu$g pro ml Carbonatpuffer (pH 8,0)
gefüllt und 4 Stunden bei Raumtemperatur stehengelassen. Danach wurden die Röhrchen entleert und zweimal mit detergenzhaltigem Phosphatpuffer gewaschen.

b) Inkubation mit h-IgE Eine Lösung von h-IgE wurde durch Zusatz von Phosphatpuffer (mit 1 % Rinderserumalbumin) auf Konzentrationen von 1 - 250 ng/ml verdünnt. Jeweils 1 ml
dieser Lösung wurde in die mit a-h-IgE beschichteten
Röhrchen gegeben und 6 Stunden bei Raumtemperatur
stehenlassen. Danach wurden die Röhrchen entleert und
zweimal mit detergenzhaltigem Phosphatpuffer gewaschen.

Die Inkubation mit Galactoseoxidase erfolgte analog
Beispiel 8 c bei einer Inkubationstemparatur von
10°C.

c) Nachweisreaktion

Die Röhrchen wurden mit 1 ml einer Reaktionslösung
analog Beispiel 1 d versetzt. Nach 10 Minuten wurde
die Extinktion der Lösung bei 492 nm gegen den Nullwert im Photometer gemessen. Wie die nachstehende Tabelle zeigt, korreliert die Extinktion mit der
Menge an zugesetztem h-IgE.

| Menge an h-IgE [ng/ml] | Extinktion bei 492 nm [OD] |
|---|---|
| 1 | 0,276 |
| 10 | 0,396 |
| 100 | 0,756 |
| 250 | 1,458 |

Merck Patent GmbH

6100 D a r m s t a d t

Patentansprüche

1. Verfahren zur Bestimmung einer Komponente aus einer Gruppe bestehend aus spezifisch bindenden Rezeptoren und Substanzen, die von diesen Rezeptoren spezifisch gebunden werden können, durch Messung der Aktivität von durch Inkubation daran gebundenen Enzymen, dadurch gekennzeichnet, daß man eine der Komponenten nach Immobilisierung an einem wasserunlöslichen Träger mit der jeweils anderen Komponente inkubiert, anschließend nach üblichem Waschen mit an Saccharid bindenden Enzymen inkubiert und die Enzymaktivität bestimmt.

2. Verfahren nach Anspruch 1, dadurch gekennzeichnet, daß als an Saccharid bindende Enzyme solche mit Lectineigenschaften bzw. Lectine mit enzymatischer Aktivität verwendet werden.

3. Verfahren nach den Ansprüchen 1 und 2, dadurch gekennzeichnet, daß als an Saccharid bindende Enzyme Galactoseoxidase, Galactosidase und/oder Mannosidase verwendet werden.

4. Verfahren nach den Ansprüchen 1 - 3, dadurch gekennzeichnet, daß als immobilisierte Komponente eine modifizierte Komponente verwendet wird, deren Saccharidgruppierung so verändert ist, daß sie keine an Saccharid bindenden Enzyme und/oder Lectine mehr bindet.

5. Verfahren nach den Ansprüchen 1 - 4, dadurch gekennzeichnet, daß man eine an einem wasserunlöslichen Träger immobilisierte Komponente gleichzeitig oder nacheinander mit der jeweils anderen Komponente und einem Kupplungsprodukt aus der jeweils anderen Komponente und einem Saccharid-haltigen Molekül inkubiert.

6. Verfahren nach den Ansprüchen 1 - 4, dadurch gekennzeichnet, daß man eine an einem wasserunlöslichen Träger immobilisierte Komponente mit der jeweils anderen, zu bestimmenden Komponente inkubiert und anschließend nach üblichem Waschen mit einer weiteren Saccharid-haltigen, an die zu bestimmende Komponente bindenden Komponente inkubiert.

7. Verfahren nach den Ansprüchen 1 - 6, dadurch gekennzeichnet, daß man die Aktivität des gebundenen Enzyms bzw. Lectins bestimmt.

8. Verfahren nach den Ansprüchen 1- 6, dadurch gekennzeichnet, daß man die Aktivität des Enzyms bzw. Lectins in der nach der letzten Inkubation erhaltenen Lösung bestimmt.

GSPHA18 A

9.   Mittel zur Durchführung des Verfahrens nach den Ansprüchen 1 - 8, enthaltend einen wasserunlöslichen
Träger, an den eine der Komponenten oder ein Komplex
aus den Komponenten, wovon eine der Komponenten
aus einem Kupplungsprodukt mit einem Saccharid
besteht, gebunden ist - gegebenenfalls ein Kupplungsprodukt aus einer der Komponenten und einem
Saccharid-haltigen Molekül - ein an Saccharid
bindendes Enzym und Substrate zur Bestimmung der
Enzymaktivität.

10.   Mittel nach Anspruch 9, dadurch gekennzeichnet,
daß der wasserunlösliche Träger ein Kunststoffgefäß ist.

11.   Mittel nach den Ansprüchen 9 und 10, dadurch gekennzeichnet, daß der wasserunlösliche Träger ein
Teströhrchen aus Polystyrol oder Polypropylen ist.

GSPHA18 A

Europäisches
Patentamt

**EUROPÄISCHER RECHERCHENBERICHT**

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| E | DE - A - 2 951 678 (E.Y. LABORA-TORIES) <br><br> * Ansprüche 1-3,8-10; Seite 12, Zeilen 13-27; Seite 13, Zeilen 1-13; Seite 15, Zeilen 17-27; Seite 16, Zeilen 1-28; Seite 17, Zeilen 1-5; Beispiel 3 * <br><br> -- | 1,2,7, 8 | G 01 N 33/54 33/58 |
| E | DE - A - 3 003 301 (OTSUKA PHARMA-CEUTICAL) <br><br> * Ansprüche 1-3; Seite 8, Zeilen 14-21; Seite 10, Zeilen 26-31; Seite 11, Zeilen 1-10 * <br><br> -- | 1,2,7, 8 | **RECHERCHIERTE SACHGEBIETE (Int. Cl.³)** |
| E | DE - A - 2 952 373 (E.Y. LABORA-TORIES) <br><br> * Ansprüche 1,5-7; Seite 11, Zeilen 8-26; Seite 12, Zeilen 1,2; Seite 13, Zeilen 15-22 * <br><br> -- | 1,2,7, 8 | G 01 N 33/54 33/58 33/76 |
| E | WO - A - 80/02296 (MASSACHUSETTS INSTITUTE OF TECHNOLOGY) <br><br> * Seite 4, Zeilen 1-20; Ansprüche 1,3 * <br><br> -- | 1,2 | **KATEGORIE DER GENANNTEN DOKUMENTE** |
| A | CHEMICAL ABSTRACTS, Band 85, Nr. 25, 20. Dezember 1976, Seite 380, Nr. 190469k <br> Columbus, Ohio, U.S.A. <br> J.K. WELTMAN et al.: "A galactosi-dase immunosorbent test for human immunoglobulin E." <br><br> & J. ALLERGY CLIN. IMMUNOL. 1976, 58(3), 426-431 <br><br> * Zusammenfassung * <br><br> -- ./. | 3 | X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument <br> &: Mitglied der gleichen Patent-familie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 05-05-1981 | DE LUCA |

EPA form 1503.1  06.78

## Europäisches Patentamt

## EUROPÄISCHER RECHERCHENBERICHT

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 90, Nr. 5, 29. Januar 1979, Seite 231, Nr. 35971c Columbus, Ohio, U.S.A. M. KIKUTANI et al.: "Enzyme immunoassay of human chorionic gonadotropin employing $\beta$-galactosidase as label" & J. CLIN. ENDOCRINOL. METAB. 1978, 47(5), 980-984 * Zusammenfassung * | 3 | |
| A | CHEMICAL ABSTRACTS, Band 90, Nr. 4, 23. Juni 1979, Seite 295, Nr. 182692r Columbus, Ohio, U.S.A. H. FRANZ et al.: "Combination of immunological and lectin reactions in affinity histochemistry: proposition of the term affinitin" & HISTOCHEMISTRY 1979, 59(4), 335-342 * Zusammenfassung * | 1 | RECHERCHIERTE SACHGEBIETE (Int. Cl. 3) |

EPA Form 1503.2   06.78